# EUROPÄISCHE PATENTSCHRIFT

(11) **EP 0 554 679 B1**
(45) Veröffentlichungstag und Bekanntmachung des Hinweises auf die Patenterteilung: **11.09.1996**
(21) Anmeldenummer: 93100445.1
(22) Anmeldetag: 14.01.1993
(51) Int. Cl.: C07C 45/46, C07C 49/80

(54) **Verfahren zur Herstellung von alpha-Chlor- 2,4,6-trimethylacetophenon**
Process for the preparation of alpha-Chloro 2,4,6-trimethylacetophenone
Procédé pour la préparation d'acetophenone alpha chlorée- 2,4,6-triméthylée

(30) Priorität: 30.01.1992 DE 4202567
(43) Veröffentlichungstag der Anmeldung: 11.08.1993
(73) Patentinhaber: BASF Aktiengesellschaft, 67063 Ludwigshafen (DE)
(72) Erfinder: Siegel, Wolfgang, Dr., W-6800 Mannheim 1 (DE); Kropp, Rudolf, W-6703 Limburgerhof (DE); Schroeder, Jochen, Dr., 67117 Limburgerhof (DE)

(56) Entgegenhaltungen:
- EP-A- 0 046 194
- EP-A- 0 411 252
- CHEMICAL ABSTRACTS, vol. 86, 1977, Columbus, Ohio, US; abstract no. 189401w,
- CHEMICAL ABSTRACTS, vol. 102, 1985, Columbus, Ohio, US; abstract no. 63988y,

## Beschreibung

Die vorliegende Erfindung betrifft ein Verfahren zur Herstellung von α-Chlor-2,4,6-trimethylacetophenon aus 1,3,5-Trimethylbenzol.

Aus der DE-A-40 00 238 ist bekannt, daß sich 2,4,6-Trimethylacetophenon aus 1,3,5-Trimethylbenzol und Acetylchlorid in Gegenwart von Aluminiumchlorid als Katalysator herstellen läßt. Nachteilig sind dabei die erforderliche molare Menge an Aluminiumtrichlorid sowie der notwendige zweifache Überschuß an 1,3,5-Trimethylbenzol.

Die Verwendung von katalytischen Mengen an Eisen(III)-Verbindungen bei Friedel-Crafts-Acylierungen mit Chloracetylchlorid ist z.B. aus J. Org. Chem. USSR 5 (1969) 1103 für FeCl₃, aus Deposited Doc. (1973) VINITI 6940-6973 für Fe₂(SO₄)₃, aus Deposited Doc. (1976) VINITI 3747-3776 und Deposited Doc. (1973) VINITI 7429-7473 für Eisenacetonylacetonat und Eisensalicylat bekannt. Eine gute Ausbeute an Acylierungsprodukt wird jedoch nur mit einem 2- bis 4-fachen Überschuß an Aromat erzielt.

Der vorliegenden Erfindung lag daher die Aufgabe zugrunde, den zuvor genannten Nachteilen abzuhelfen.

Demgemäß wurde ein neues und verbessertes Verfahren zur Herstellung von α-Chlor-2,4,6-trimethylacetophenon der Formel I 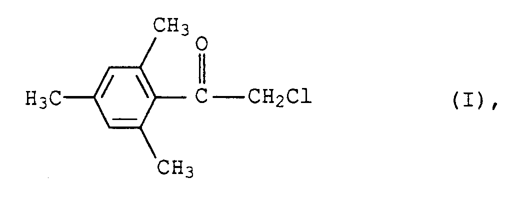 durch Umsetzung von 1,3,5-Trimethylbenzol mit Chloracetylchlorid in Gegenwart eines Katalysators bei Temperaturen von 0 bis +150°C und Drücken von 0,01 bis 50 bar durchführt, gefunden, welches dadurch gekennzeichnet ist, daß man als Katalysator Eisenoxide verwendet.

Das erfindungsgemäße Verfahren läßt sich wie folgt durchführen:

Die Umsetzung von 1,3,5-Trimethylbenzol und Chloracetylchlorid kann bei Temperaturen von 0 bis 150°C, bevorzugt 50 bis 100°C, besonders bevorzugt 70 bis 90°C und Drücken von 0,01 bis 50 bar, bevorzugt 0,1 bis 5 bar, besonders bevorzugt bei Normaldruck (Atmosphärendruck) in Gegenwart von 0,0001 bis 5 Mol-%, bevorzugt 0,001 bis 2 Mol-%, besonders bevorzugt 0,05 bis 0,5 Mol-% eines Eisenoxids, bevorzugt Eisen(III)oxid als Katalysator durchgeführt werden.

Das Molverhältnis von Chloracetylchlorid zu 1,3,5-Trimethylbenzol liegt in der Regel bei 0,8 : 1 bis 5 : 1, bevorzugt 0,9 : 1 bis 2,5 : 1, besonders bevorzugt 1 : 1 bis 2 : 1.

Die Reaktion kann in Gegenwart oder in Abwesenheit eines inerten Lösungsmittels durchgeführt werden. Als inerte Lösungsmittel eignen sich aromatische Lösungsmittel wie Nitrobenzol, Chlorbenzol, und aliphatische Lösungsmittel wie Heptan, Toluol, Benzol und chlorierte Lösungsmittel wie Methylchlorid, Mesitylen, bevorzugt Chlorbenzol, Heptan und Mesitylen, besonders bevorzugt Chlorbenzol und Mesitylen.

Das α-Chlor-2,4,6-trimethylacetophenon I eignet sich als Vorprodukt zur Herstellung von 2,4,6-Trimethylbenzoesäure, das ein wertvoller Ausgangsstoff für Farbstoffe, Schädlingsbekämpfungsmittel und Pharmazeutika und Photoinitiatoren ist. Beispielsweise ergibt das der 2,4,6-Trimethylbenzoesäure entsprechende Benzoylchlorid durch Umsetzung mit Alkoxyphosphinen Verbindungen, die als wertvolle Photoinitiatoren verwendet werden.

Bezüglich weiterer Verwendung wird auf die vorgenannten Veröffentlichungen und Ullmanns Encyclopädie der technischen Chemie, Band 4, Seiten 272 bis 291, verwiesen.

### Beispiele

### Beispiel 1

240 g 1,3,5-Trimethylbenzol und 80 mg Eisen(III)oxid und 240 ml Chlorbenzol werden vorgelegt und zum Rückfluß (ca. 120°C) erhitzt. Innerhalb einer Stunde werden 230 g Chloracetylchlorid zugegeben. Nach kurzer Zeit setzt HCl-Entwicklung ein. Man rührt nach beendeter Zugabe noch zwei Stunden nach, bis die Gasentwicklung beendet ist. Die so erhaltene Lösung wird ohne Aufarbeitung in die Haloformspaltung eingesetzt.

### Beispiel 2

120 g 1,3,5-Trimethylbenzol und 40 mg Eisen(III)oxid werden vorgelegt und auf 80°C aufgeheizt. Innerhalb einer Stunde werden 113 g Chloracetylchlorid zugegeben. Nach beendeter Zugabe rührt man noch 4 h nach, bis zum Ende der HCl-Entwicklung. Das Produkt wird ohne Aufarbeitung in die Haloformspaltung analog Beispiel 4 eingesetzt.

### Beispiel 3 (Weiterverarbeitung zur 2,4,6-Trimethylbenzoesäure)

500 g Natronbleichlauge (ca. 13% aktives Chlor), 720 g Natronlauge (25 %ig) und 4 g Dimethyldibenzylammoniumchlorid als 50 %ige wäßrige Lösung werden vorgelegt und auf 50°C aufgeheizt. Man gibt anschließend 202 g einer Lösung von a-Chlor-2,4,6-trimethylacetophenon in Chlorbenzol (Beispiel 1) zu. Die Temperatur wird durch Kühlen bei 50 bis 60°C gehalten. Innerhalb einer Stunde werden dann 66 g Chlorgas eingeleitet. Nach beendeter Chlorzugabe wird noch fünf Stunden auf Rückflußtemperatur (105 bis 108°C) aufgeheizt, wobei das Chlorbenzol über einen Wasserabscheider ausgekreist wird.

Die gelbe Reaktionslösung wird nach dem Abkühlen auf Raumtemperatur abgelassen und mit ca. 200 ml konzentrierter Salzsäure auf pH = 2 eingestellt. Die weiße Suspension wird abfiltriert und der Niederschlag zweimal mit je 250 ml kaltem Wasser gewaschen und im Vakuumtrockenschrank bei 80°C getrocknet. Man erhält 90,3 g (90 %) 2,4,6-Trimethylbenzoesäure (Smp.: 155°C).

### Beispiel 4 (Weiterverarbeitung zur 2,4,6-Trimethylbenzoesäure)

500 g Natronbleichlauge (ca. 13% aktives Chlor), 720 g Natronlauge (25 %ig) und 4 g Dimethyldibenzylammoniumchlorid als 50 %ige wäßrige Lösung werden vorgelegt und auf 50°C aufgeheizt. Man gibt anschließend 160 g eines Reaktionsaustrages analog Beispiel 2 zu. Die Temperatur wird durch Kühlen bei 50 bis 60°C gehalten. Innerhalb einer Stunde werden dann 66 g Chlorgas eingeleitet. Nach beendeter Chlorzugabe wird noch fünf Stunden auf Rückflußtemperatur (ca. 105 bis 108°C) aufgeheizt.

Die gelbe Reaktionslösung wird nach dem Abkühlen auf Raumtemperatur abgelassen und mit ca. 200 ml konzentrierter Salzsäure auf pH = 2 eingestellt. Die weiße Suspension wird abfiltriert und der Niederschlag zweimal mit je 250 ml kaltem Wasser gewaschen und im Vakuumtrockenschrank bei 80°C getrocknet. Man erhält 123,8 g (87 %) 2,4,6- Trimethylbenzoesäure (Smp.: 155°C).

## Patentansprüche

1. Verfahren zur Herstellung von α-Chlor-2,4,6-trimethylacetophenon der Formel I 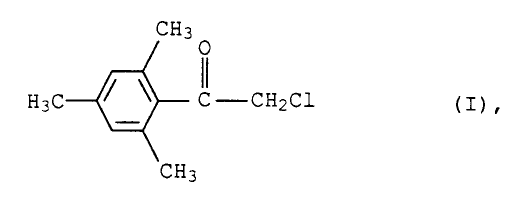 durch Umsetzung von 1,3,5-Trimethylbenzol mit Chloracetylchlorid in Gegenwart eines Katalysators bei Temperaturen von 0 bis +150°C und Drücken von 0,01 bis 50 bar, dadurch gekennzeichnet, daß man als Katalysator Eisenoxide verwendet.

2. Verfahren zur Herstellung von α-Chlor-2,4,6-trimethylacetophenon nach Anspruch 1, dadurch gekennzeichnet, daß man als Katalysator Eisen-(III)-oxid verwendet.

3. Verfahren zur Herstellung von α-Chlor-2,4,6-trimethylacetophenon nach Anspruch 1 und 2, dadurch gekennzeichnet, daß man 1,3,5-Trimethylbenzol und Chloracetylchlorid im Molverhältnis von 0,3 : 1 bis 3 : 1 einsetzt.

4. Verfahren zur Herstellung von α-Chlor-2,4,6-trimethylacetophenon nach Anspruch 1 und 2, dadurch gekennzeichnet, daß man 1,3,5-Trimethylbenzol und Chloracetylchlorid im Molverhältnis von 0,8 : 1 bis 1,2 : 1 einsetzt.

## Claims

1. A process for preparing α-chloro-2,4,6-trimethylacetophenone of the formula I 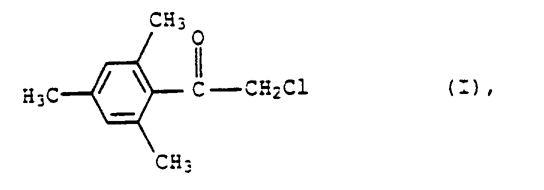 by reacting 1,3,5-trimethylbenzene with chloroacetyl chloride in the presence of a catalyst at from 0 to +150°C under from 0.01 to 50 bar, wherein iron oxides are used as catalyst.

2. A process for preparing α-chloro-2,4,6-trimethylacetophenone as claimed in claim 1, wherein iron(III) oxide is used as catalyst.

3. A process for preparing α-chloro-2,4,6-trimethylacetophenone as claimed in claim 1 and 2, wherein 1,3,5-trimethylbenzene and chloroacetyl chloride are used in the molar ratio of from 0.3:1 to 3:1.

4. A process for preparing α-chloro-2,4,6-trimethylacetophenone as claimed in claim 1 and 2, wherein 1,3,5-trimethylbenzene and chloroacetyl chloride are used in the molar ratio of from 0.8:1 to 1.2:1.

## Revendications

1. Procédé de préparation de l'α-chloro-2,4,6-tétraméthylacétophénone de la formule I 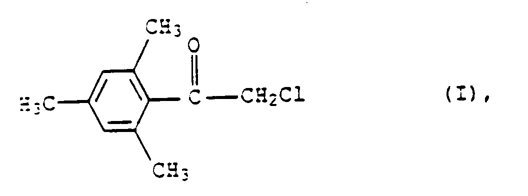 par la réaction du 1,3,5-triméthylbenzène avec le chlorure de chloracétyle en présence d'un catalyseur à des températures de 0 à +150°C et sous des pressions de 0,01 à 50 bars, caractérisé en ce que l'on utilise des oxydes de fer à titre de catalyseur.

2. Procédé de préparation de l'α-chloro-2,4,6-triméthylactophénone suivant la revendication 1, caractérisé en ce que l'on utilise l'oxyde de fer-(III) à titre de catalyseur.

3. Procédé de préparation de l'α-chloro-2,4,6-triméthylacétophénone suivant l'une quelconque des revendications 1 et 2, caractérisé en ce que l'on met en oeuvre le 1,3,5-triméthylbenzène et le chlorure d'acétyle dans le rapport molaire de 0,3:1 à 3:1.

4. Procédé de préparation de l'α-chloro-2,4,6-triméthylacétophénone suivant l'une quelconque des revendications 1 et 2, caractérisé en ce que l'on met en oeuvre le 1,3,5-triméthylbenzène et le chlorure de chloracétyle dans le rapport molaire de 0,8:1 à 1,2:1.
